(19) Europäisches Patentamt
European Patent Office
Office européen des brevets

(11) EP 1 254 120 B1

(12) EUROPEAN PATENT SPECIFICATION

(45) Date of publication and mention of the grant of the patent:
**14.07.2004 Bulletin 2004/29**

(21) Application number: **01902578.2**

(22) Date of filing: **06.02.2001**

(51) Int Cl.[7]: **C07D 239/52**, A61K 31/505

(86) International application number:
**PCT/IB2001/000142**

(87) International publication number:
**WO 2001/058882 (16.08.2001 Gazette 2001/33)**

# (54) USE OF PYRIMIDINE ENDOTHELIN ANTAGONISTS IN COMPANION ANIMALS

VERWENDUNG VON PYRIMIDIN-ENDOTHELIN-ANTAGONISTEN IN HAUS- UND NUTZTIEREN

UTILISATION DE PYRIMIDINES ANTAGONISTES DE L'ENDOTHELINE CHEZ LES ANIMAUX FAMILIERS

(84) Designated Contracting States:
**AT BE CH CY DE DK ES FI FR GB GR IE IT LI LU MC NL PT SE TR**

(30) Priority: **11.02.2000 GB 0003234**

(43) Date of publication of application:
**06.11.2002 Bulletin 2002/45**

(73) Proprietors:
- **Pfizer Limited**
  **Sandwich, Kent CT13 9NJ (GB)**
  Designated Contracting States:
  **GB**
- **PFIZER INC.**
  **New York, N.Y. 10017 (US)**
  Designated Contracting States:
  **AT BE CH CY DE DK ES FI FR GR IE IT LU MC NL PT SE TR**

(72) Inventors:
- **BANKS, Bernard J.,**
  **Pfizer Global Research and Dev.**
  **Sandwich, Kent CT13 9NJ (GB)**
- **FOSTER, Adrian P.,**
  **Pfizer Global Research and Dev.**
  **Sandwich, Kent CT13 9NJ (GB)**
- **GIBSON, Stephen P,**
  **Pfizer Global Research and Dev.**
  **Sandwich, Kent CT13 9NJ (GB)**

(74) Representative: **Atkinson, Jonathan David Mark**
**Urquhart-Dykes & Lord LLP**
**Tower North Central**
**Merrion Way**
**Leeds LS2 8PA (GB)**

(56) References cited:
**EP-A- 0 882 719** **WO-A-98/57938**

EP 1 254 120 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

## Description

This invention relates to the use of certain endothelin antagonists in the treatment of companion animals suffering from conditions mediated by endothelin.

Endothelin (ET) is a potent vasoconstrictor synthesised and released by endothelial cells. There are three distinct isoforms of ET: ET-1, ET-2 and ET-3, all being 21-amino acid peptides and herein the term 'endothelin' refers to any or all of the isoforms. Two receptor subtypes, $ET_A$ and $ET_B$ have been pharmacologically defined (see for example H. Arai et al, Nature, 348, 730 , 1990) and further subtypes have recently been reported. Stimulation of $ET_A$ promotes vasoconstriction, and stimulation of $ET_B$ receptors causes either vasodilation or vasoconstriction. The main effects of ET are observed in the cardiovascular system, particularly in the coronary, renal, cerebral and mesenteric circulation, and the effects of endothelin are often long-lasting. Stimulation of ET receptors also mediate further biological responses in cardiovascular and non-cardiovascular tissues such as cell proliferation and matrix formation.

Increased circulating levels of endothelin have been observed in patients who have undergone percutaneous transluminal coronary angioplasty (PTCA) (A.Tahara et al, Metab. Clin. Exp. 40, 1235, 1991) and ET-1 has been found to induce neointimal formation in rats after balloon angioplasty (S.Douglas et al, J.Cardiovasc.Pharm., 22 (Suppl 8), 371, 1993). The same workers have found that an endothelin antagonist, SB-209670, causes a 50% reduction in neointimal formation relative to control animals (S.Douglas et al, Circ Res, 75, 1994). Antagonists of the endothelin receptor may thus be useful in preventing restenosis post PTCA. The $ET_{A/B}$ receptor antagonist Bosentan reportedly decreased blood pressure in hypertensive patients (New Eng.J.Med. (1998) 338, 784-790). Antagonists of $ET_B$ receptors such as BQ-788 have been demonstrated to increase peripheral resistance in man (Hypertension (1999) 33, 581-585). Thus $ET_A$-selective receptor antagonists are likely to be of benefit in hypertension.

Endothelin-1 is produced in the human prostate gland and endothelin receptors have been identified in this tissue (Eur. J.Pharmacol. (1988) 349, 123-128). Since endothelin is a contractile and proliferative agent, endothelin antagonists could be useful in the treatment of benign prostate hypertrophy.

There is widespread localisation of endothelin and its receptors in the central nervous system and cerebrovascular system (R.K.Nikolov et al, Drugs of Today, 28(5), 303, 1992) with ET being implicated in cerebral vasospasm, cerebral infarcts, septic shock, myocardial infarction and neuronal death.

Elevated levels of endothelin have also been observed in patients with:

- recurrent airway obstruction (Pulm.Pharm.Ther. (1998) 11: 231-235);
- asthma (Am.J.Resp.Crit. Care Med. (1995) 151:1034-1039);
- acute renal failure (K.Tomita, et al, Med.Philos. (1994) 13(1), 64-66);
- chronic renal failure (F.Stockenhuber et al, Clin Sci (Lond.), 82, 255, 1992);
- ischaemic Heart Disease (M.Yasuda, Am. Heart J., 119, 801, 1990);
- stable or unstable angina (J.T.Stewart, Br. Heart J. 66, 7 1991);
- pulmonary hypertension (D.J.Stewart et al, Ann. Internal Medicine, 114, 464, 1991 );
- congestive heart failure (R.J.Rodeheffer et al, Am.J.Hypertension, 4, 9A, 1991 );
- preeclampsia (B.A.Clark et al, Am.J.Obstet.Gynecol., 166, 962, 1992);
- diabetes (A.Collier et al, Diabetes Care, 15 (8), 1038, 1992);
- Crohn's disease (S.H.Murch et al, Lancet, 339, 381, 1992); and
- atherosclerosis (A.Lerman et al, New Eng. J. Med., 325, 997, 1991).

Such diseases may be referred to as "endothelin-mediated disorders". Although the above discussion relates mainly to humans, corresponding disease states may be found in companion animals such as dogs, cats and horses. In every case the disease state associated with the physiologically elevated levels of endothelin is potentially treatable with a substance which decreases the effect of endothelin, such as an endothelin receptor antagonist, or a compound which binds endothelin such that it reduces the effective concentration thereof at the endothelin receptors.

It has now been found that a small group of endothelin receptor antagonist compounds are particularly useful in the treatment of endothelin-mediated disorders in companion animals (such as dogs, cats and horses).

Thus, according to the present invention, there is provided the use of a compound of formula I,

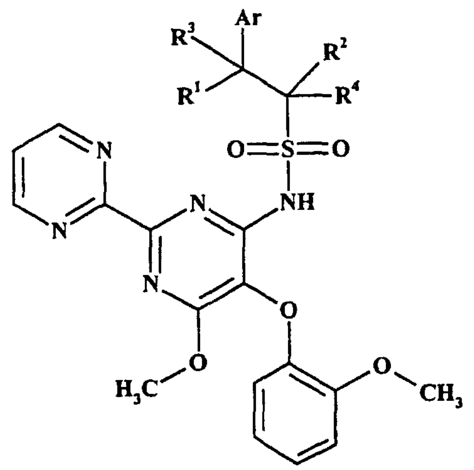

I

wherein $R^1$ and $R^2$ each represent H, or together represent a second carbon-carbon bond between the carbon atoms to which they are attached;

when $R^1$ and $R^2$ each represent H, then $R^3$ and $R^4$ also represent H;

when $R^1$ and $R^2$ together represent a second carbon-carbon bond between the carbon atoms to which they are attached, then $R^3$ and $R^4$ independently represent H or $C_{1-6}$ alkyl;

Ar represents:

phenyl or naphthyl, which groups are optionally substituted by one or more groups selected from $C_{1-6}$ alkyl [which may itself be substituted by one or more substituents selected from halo, $C_{1-6}$ alkoxy, $CO_2H$, $NH_2$, $NH(C_{1-6}$ alkyl) and $N(C_{1-6}$ alkyl$)_2$], halo, $C_{1-6}$ alkoxy, $CO_2H$, $C_{1-6}$ alkoxycarbonyl, $NO_2$, CN, $NH_2$, $NH(C_{1-6}$ alkyl), $N(C_{1-6}$ alkyl$)_2$, OH and $C_{1-3}$ alkylenedioxy, or

a 5- or 6-membered heteroaryl ring containing up to 4 heteroatoms selected from N, O and S, which group is optionally substituted by one or more groups selected from $C_{1-6}$ alkyl [which may itself be substituted by one or more substituents selected from halo, $C_{1-6}$ alkoxy, $CO_2H$, $NH_2$, $NH(C_{1-6}$ alkyl) and $N(C_{1-6}$ alkyl$)_2$], halo, $C_{1-6}$ alkoxy, $CO_2H$, $C_{1-6}$ alkoxycarbonyl, $NO_2$, CN, $NH_2$, $NH(C_{1-6}$ alkyl) and $N(C_{1-6}$ alkyl$)_2$;

or a veterinarily acceptable salt thereof;

in the manufacture of a medicament for the treatment or prophylaxis of an endothelin-mediated disorder in a companion animal.

Veterinarily acceptable salts include alkali and alkaline earth metal salts (for example sodium and potassium salts), and salts formed with basic amines (for example, tri($C_{1-6}$ alkyl)-substituted amines).

Preferred features of the invention include:

(a) the companion animal is a cat, a dog or a horse (more preferably a dog or cat; most preferably a dog);

(b) the endothelin-mediated disorder is hypertension, congestive heart failure or chronic renal failure;

(c) $R^1$ and $R^2$ each represent H;

(d) $R^3$ and $R^4$ each represent H; and

(e) Ar represents phenyl, naphthyl or thienyl, which groups are optionally substituted by one or more groups selected from $C_{1-6}$ alkyl, halo, $CF_3$, $C_{1-6}$ alkoxy, $CO_2H$ and $C_{1-6}$ alkoxycarbonyl - most preferably, Ar is phenyl.

Of special interest is the treatment of congestive heart failure in dogs.

Of further special interest is the treatment of chronic renal failure in cats.

Compounds of formula I in which $R^1$ and $R^2$ each represent H are disclosed in International Patent Application WO 98/57938. Compounds of formula I in which $R^1$ and $R^2$ together represent a second carbon-carbon bond between the carbon atoms to which they are attached are disclosed in European Patent Application 882719. Both of these publications are herein incorporated by reference.

The compound of formula I in which $R^1$, $R^2$, $R^3$ and $R^4$ each represent H, and Ar is phenyl is N-[6-methoxy-5-(2-methoxyphenoxy)-2-(2-pyrimidinyl)-4-pyrimidinyl]-2-phenylethansulphonamide. It is disclosed in Example 1 of International Patent Application WO 98/57938, is referred to herein as "Compound X", and is of particular interest.

The compound of formula I in which $R^1$ and $R^2$ together represent a second carbon-carbon bond between the carbon atoms to which they are attached, $R^3$ and $R^4$ each represent H, and Ar is phenyl is N-[6-methoxy-5-(2-methoxyphenoxy)-2-(2-pyrimidinyl)-4-pyrimidinyl]-2-phenylethensulphonamide. It is disclosed in Example 2 of European Patent Application 882719, is referred to herein as "COMPOUND Y", and is of particular interest.

The invention has the advantage that the compounds of formula I have a longer duration of action than the compounds of the prior art. This means that the frequency with which they must be administered will be reduced. This is particularly important with veterinary medicines, because animals often resist frequent administration of medicaments. Therefore, the reduced administration frequency will lead to more regular treatment (enhancing disease control) and greater convenience (enhancing patient compliance). The biological activity of the compounds of formula I (and some comparator compounds) was assessed in the following tests.

Test A

Inhibition of contraction induced by ET-1 in dog renal artery strips to generate $K_b$ values

A $K_b$ value is a measure of functional activity, and this is taken to be the free plasma concentration of a test compound required to provide efficacy.

Renal arteries from beagle dogs (body weight 9-15 kg) were cut into spirals and divided into 5 mm long strips. The side of each strip that was adjacent to the lumen of the vessel was gently rubbed to remove the endothelium. Each strip was mounted under a resting tension of 1.2g in an organ bath containing Krebs-Henseleit solution at 37°C gassed with 5% $CO_2$ in 95% $O_2$. The tension generated by each strip was recorded isometrically. After incubating the tissues with test compound for 75 minutes, cumulative concentration response curves were constructed to ET-1 in the presence of 10μM Bestatin, 10μM Captopril, 10μM Thiorphan, 50μM indomethacin and 0.001% BSA. Only a single ET-1 curve was constructed in each tissue and tension increases expressed as a percentage of the response to 120mM KCI determined before the addition of the test compound. The antagonistic activity of the test compound was determined by calculating the apparent $K_b$ for a single concentration of each compound using the method described by MacKay (J. Pharm. Pharmac. 30, 321-313, 1978). The concentration of ET-1 causing 50% of the maximum response ($EC_{50}$) was calculated and from these the dose ratio (DR). This is the ratio of the $EC_{50}$ in the presence of the test compound divided by the EC50 in its absence. The DR is then put into the following equation:

$$\text{apparent } pK_b = \log(DR-1) - \log[B]$$

(where B = molar concentration of test compound)

$$\text{apparent } K_b = \text{antilog } pK_b$$

Results

| Compound | Apparent $K_b$ (nM) |
|---|---|
| COMPOUND Y | 58 |
| LU 135,252[(1)] | 111 |
| Example 2, WO 98/57938[(2)] | 136 |
| Compound X | 86 |

(1) Structure of LU 135,252 (comparator compound) is:

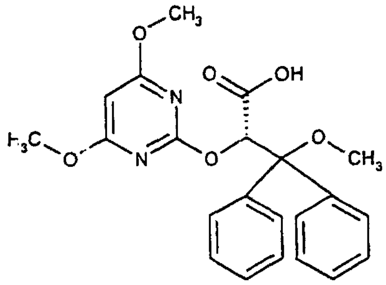

(2) Structure of Example 2, WO 98/57938 (comparator compound) is:

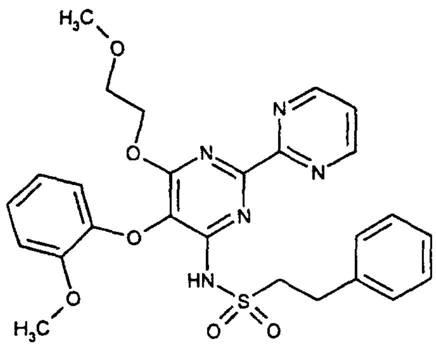

Test B

Pharmacokinetic studies

In order to assess the potential of test ET antagonists to provide efficacy, a pharmacokinetic study in the dog was carried out.

It is generally assumed that the fraction of drug that is free (i.e. unbound) in plasma is in equilibrium with free drug in other aqueous compartments of the body, provided the drug is able to cross membrane barriers. Hence, whilst the actual amount of drug in plasma may represent a small proportion of the drug in the body, it provides valuable information on the free concentration of the drug throughout the body. Assuming that only free drug is available to exert a pharmacological effect, then determining the free drug concentration in plasma and combining this with knowledge of drug potency, provides a measure of the potential efficacy of that compound in vivo [see William J. Jusko and Mark Gretch, 'Plasma and tissue protein binding of drugs in pharmacokinetics', Drug Metabolism Reviews, 5(1), 43-140 (1976)].

(a) Analysis of Plasma Samples

Test compounds were administered to a dog by i.v. infusion over 10 minutes to give a dose of 0.5 mg/kg. Blood samples were taken at various time points and centrifuged to provide plasma samples. These samples were analysed using Mass Spectrometry to provide total plasma concentrations.

Plasma samples and standards (prepared using blank plasma spiked with the appropriate concentration of compound), were typically prepared by protein precipitation with acetonitrile (MeCN) [2:1 MeCN/plasma], centrifuged and the supernatant injected into a liquid chromatography/mass spectrum/mass spectrum (LC/MS/MS) system (PE Sciex API 365) running with the following conditions:

| | |
|---|---|
| Flow rate | 1.0 ml/min |
| Column | Hypersil™ BDS C18, 5µm, 4.6mm i.d. x 50 mm. |
| Mobile Phase | (A) 5 mM ammonium acetate in 2% MeCN 98% $H_2O$ |
| | (B) 5mM ammonium acetate in 90% MeCN 10% $H_2O$ |
| Gradien | |

(continued)

| | | | | |
|---|---|---|---|---|
| | 0.0 min | 95% (A) | 5% (B) | |
| | 1.0 min | 95% (A) | 5% (B) | |
| | 2.0 min | 5% (A) | 95% (B) | |
| | 4.0 min | 5% (A) | 95 % (B) | |
| | 6.5min | 95% (A) | 5% (B) | First 2.5 minutes diverted to waste, the remainder to MS |

| | | | |
|---|---|---|---|
| Injection volume of supernatant prepared above | 150 µl | | |
| MS Mode | Ionspray, positive ion, MRM | | |
| Dwell time. | 500 msec | Pause time | 50 msec |
| Collision gas setting | 1 | Ion energy | optimised for each compound |

Total plasma concentrations in the dog were determined by comparison with the standard line constructed (using the spiked plasma samples) to cover the expected concentration range.

Results

| Compound | Total plasma concentration at 24 hours (nM) following 0.5 mg/kg iv dose |
|---|---|
| COMPOUND Y | 1161 (25 h) |
| LU 135,252 | 40 |
| Example 2, WO 98/57938 | <93 |
| Compound X | 466 |

(b) Determination of plasma protein binding by equilibrium dialysis

The methods used are based on the principles outlined by Pacifici and Viani ['Methods of determining plasma and tissue binding of drugs', Clinical Pharmacokinetic Concepts 23 (6), 449-468 (1992)]. An example of a method used is described below.

Plasma containing the test compound was placed in a chamber (half-cell), separated by a semi-permeable membrane (Spectra/Por™ 1 47 mm diam MWCO 6-8000) from isotonic buffer at physiological pH in the other half-cell. The system was allowed to equilibrate, such that low molecular weight compounds distributed evenly between the two chambers (plasma and buffer), whilst high molecular weight molecules were restricted to the plasma chamber. At equilibrium the concentration of the unbound test compound is the same on either side of the membrane. The concentration of total test compound in each half-cell was then determined (using essentially the described above) and the extent of plasma protein binding calculated.

The Spectra/Por™ Equilibrium Dialyzer used to determine protein binding was supplied by NBS Biologicals.

| Results | |
|---|---|
| Compound | Plasma protein binding (%, canine) |
| COMPOUND Y | 87 |
| LU 135,252 | 96.8 |
| Example 2, WO 98/57938 | 88 |
| Compound X | 91 |

(c) Calculation of free plasma concentrations

$$\text{Fraction unbound (Fu)} = \frac{\text{concentration in buffer}}{\text{concentration in plasma}}$$

$$\text{or \% protein bound} = (1\text{-Fu})\text{x } 100$$

The free plasma concentrations of the compound can then be determined.

Free plasma concentration = total plasma concentration (from method (a)) x fraction unbound (from method (b))

| Results | |
|---|---|
| Compound | Free concentration at 24 hours (nM) following 0.5 mg/kg i.v. dose |
| COMPOUND Y | 151 (25 h) |
| LU 135,252 | 1.3 |
| Example 2, WO 98/57938 | <11 |
| Compound X | 41.9 |

From the above data, the free plasma concentration found may be expressed as a percentage of the free plasma concentration of the test compound required to provide efficacy ($K_b$ value, from Test A above), and by multiplying by 4, the percentage of $K_t$ may be estimated for a 2.0 mg/kg dose (a dose likely to be used in practice):

| Compound | % of $K_b$ at 24 hours following 0.5 mg/kg i.v. dose | Estimated % of $K_b$ at 24 hours following 2.0 mg/kg i.v. dose |
|---|---|---|
| COMPOUND Y | 260 | 1040 |
| LU 135,252 | 1.2 | 4.8 |
| Example 2, WO 98/57938 | <8 | <32 |
| Compound X | 49 | 196 |

From the above table, it can be seen that only COMPOUND Y and Compound X would still be efficacious after 24 hours following a 2.0 mg/kg i.v. dose (because they would be present at 100% of the $K_b$ value or more).

Test C

Inhibition of ET-1-induced pressor response in conscious beagle dogs

Studies were performed in surgically prepared conscious beagle dogs (13-17 kg) which were well adjusted to the laboratory environment. Surgical preparation was performed under recovery anaesthesia to insert an indwelling aortic catheter with external access between the scapulae and protruding subcutaneous ECG studs, positioned for the recording of lead II electrocardiograms. During studies dogs were placed, unrestrained, in "Pavlov" type slings. The aortic catheter was connected to a pressure transducer for the measurement of systemic arterial pressure and ECG studs, via patient cables, to a bio-amplifier for the recording of electrocardiograms. ET-1 was administered as a 15 min infusion (25 pmoles/kg/min) via a temporary catheter inserted into the saphenous vein. Test compound or placebo was administered by oral gavage (0.5 or 2.0 mg/kg) at 1 and 24 hours prior to the infusion of ET-1. Individual studies investigated no more than a single dose of test compound or placebo at any one time. Systemic arterial pressure and electrocardiograms were recorded for one hour prior to and one hour after the start of the infusion of ET-1. Test compounds were evaluated by comparing the effects of placebo or test compound on the magnitude of pressor response elicited by ET-1.

Oral administration of test compounds exhibited an excellent suppressive action on the ET-1-induced pressor responses in conscious dogs. The changes in arterial pressure (ΔmmHg) are shown in the following tables:

| Time | Dose | Placebo ΔmmHg | COMPOUND Y, ΔmmHg | % inhibition |
|---|---|---|---|---|
| 1 hour | 0.5 mg/kg p.o. | 18 | 7 | 57 |
| 24 hours | 2.0 mg/kg p.o. | 24 | 13 | 44 |

| Time | Dose | Placebo ΔmmHg | Compound X, ΔmmHg | % inhibition |
|---|---|---|---|---|
| 1 hour | 0.5 mg/kg p.o. | 17 | 3 | 76 |
| 24 hours | 2.0 mg/kg p.o. | 12 | 3 | 80 |

The treatment of dogs with congestive heart failure is discussed further below.

Congestive heart failure (CHF) is a complex clinical syndrome characterised by abnormalities of ventricular function and neurohumoral changes, accompanied by exercise intolerance, fluid retention and reduced survival. The main causes of CHF in companion animals (CA) are hypertension, myopathy of various aetiology and valvular dysfunction, whereas coronary artery disease, the main cause in humans plays a minor role [1]. CHF is a common disease with an age-dependent prevalence of 1-10% [2]. In CHF the presence of a correctable condition should always be determined. Unfortunately, however, the underlying pathophysiological disorder usually is not reversible by specific treatment, and only a preventive approach can be expected to have a major impact on the outcome of this disease. Nevertheless, because specific treatment is not possible in the majority of clinically ill CHF-patients the goal of the therapy envisioned herein is to ameliorate symptoms and to delay progressive myocardial damage to reduce mortality and to improve quality of life for the pet and its owner.

The clinical syndrome of CHF is dominated by left ventricular dysfunction, and drug therapy is often focused on whether reversal of left ventricular dysfunction can improve both symptoms and prognosis.

Current therapy relies largely on diuretics (ca. 70% of cases with dilated cardiomyopathy) and digoxin (ca. 61% of cases). This dual therapy regime does not significantly improve the clinical outcome of dogs with heart disease since treatment is still associated with an overall poor prognosis (survival rates of ca. 17.5% at one year and ca. 7.5% at two years [3]). Veterinarians, therefore, do sometimes employ ACE inhibition to treat heart disease in dogs (<10% of cases, ref 3) and a recent veterinary clinical trial revealed a significant slow down in disease progression by enalapril [4], but did not improve cardiac function. There is scope therefore for an improved treatment which improves cardiac function.

Endothelin (ET)-1, an endothelium derived peptide can cause not only potent and prolonged vasoconstriction but also sodium retention and activation of catecholamine, angiotensin II and aldosterone secretion. These effects may significantly contribute to development and progression of CHF [5]. Significant elevations of plasma ET-1 levels are observed in human CHF and these values are strongly related to survival in CHF patients [6]. The study we carried out included both haemodynamic and clinical endpoints and was designed to come as close to the clinical situation as possible.

A group of animals receiving ACE-inhibitor treatment served as positive control.

Heart failure was induced by rapid ventricular pacing, which is a well established and extensively documented model [2,9]. It is the only heart failure model that allows control of the degree of heart failure by adjusting the pacing rate. It has been shown to be suitable for the planned experiments in previous pharmacological work in many studies by other groups using closely related substance classes [7,10].

The effects of drugs during the stages of heart failure that precede severe clinical illness at rest were measured. According to the classification by the New York Heart Association (NYHA), which is commonly used in veterinary practice [4], CHF is subdivided into 4 clinical stages according to the following criteria:

Stage I: Normal activity does not produce undue fatigue, dyspnoea or coughing (only dogs). Exercise capacity is reduced.

Stage II: The dog/cat is comfortable at rest but ordinary physical activity causes fatigue, dyspnoea or coughing (see above).

Stage III: The dog/cat is comfortable at rest, but minimal exercise produces fatigue or dyspnoea

Stage IV: Decompensated heart failure. Dyspnoea is present even at rest, absolute EI.

Dogs were paced throughout the study at pacing rates up to 220-240 bpm leading to steadily progressing CHF.

Treatment with either

Group 1: Placebo (Furosemide 4 mg/kg/day BID per os, n=6)
Group 2: ET-receptor antagonist (2 mg/kg/day SID per os + Furosemide as above, n=6)
Group 3: Enalapril maleate (1 mg/kg/day BID per os + Furosemide as above) (n=6, positive control)

was initiated 2 weeks after start of pacing and continued for 3 weeks.
Throughout the study, both clinical parameters (heart murmurs, edema formation, dyspnoe) as well as haemodynamic (CO, blood pressure, cardiac contractility, and heart rate) and humoral parameters (plasma catecholamines, ANF, ET-1) were measured to determine morbidity and quality of life of the animals.

References

1 Detweiler D.K. and Patterson D.F. (1986) The prevalence and types of cardiovascular disease in dogs. Ann. N. Y. Acad. Sci. 481-516.
2 Pensinger R.R. (1986) Comparative aspects of congestive heart failure in dogs. 335-345.
3 Tilholm A., Svensson H. and Sylven C. (1997) Survival and prognostic factors in 189 dogs with dilated cardiomyopathy. J. Am. An. Hosp. Assoc. 33, 364-368.
4. Ettinger, S.J. et al. (1998) Effects of enalapril maleate on survival of dogs with naturally acquired heart failure. JAVMA 213, 1573-1577
5. Miller, WL et al. (1989) Integrated cardiac, renal and endocrine actions of endothelin. J Clin Invest 83, 317-320
6. Pacher, R. et al. (1996) Prognostic impact of big endothelin-1 plasma concentrations compared with invasive hemodynamic evaluation in severe heart failure. Am J Coll Cardiol 27, 633-641
7. Moe, GW et al. (1998) Beneficial effects of long-term selective endothelin type A receptor blockade in canine experimental heart failure. Cardiovasc Res 39, 571-579
8. Coleman, H.N. et al. (1971) Congestive heart failure following chronic tachycardia. Am Heart J 81: 790-798
9. Hasenfuss, G. (1998) Animal models of cardiovascular disease, heart failure and hypertrophy Cardiovasc Res 39: 60-76
10. Sato, N. et al. (1999) Mechanisms of desensitization to a PDE-inhibitor (milrinone) in conscious dogs with heart failure Am J Physiol 276: H1699-H1705

Protocol:

1. Treadmill training

During the experiments the dogs had to run (trot) voluntarily (for up to 20 min) on a treadmill during continuous measurement of heamodynamic parameters (cables to implants connected).
To assure training success, only dogs that were shown to run 3 times for at least five minutes successfully on the treadmill, were selected for the study. Conversely, animals that do not run on the treadmill after 3 sessions were considered as training failures and excluded from the study.
All dogs that successfully completed training were re-examined by a veterinary surgeon and declared suitable for use. The examination included, at the discretion of veterinary surgeon, repeated blood sampling for haematology and clinical chemistry.
Trained dogs (3 x 2/week = 6 animals first) were subjected to a 2 week pre-surgery period. During this time the animals were familiarised with experimenters and the Lab. Further treadmill training sessions (minimum 2) were carried out to ensure readiness for the study.

2. Surgery

The dogs were instrumented with the following using standard surgical tecnhiques:

- Pulmonary artery flow probe (Triton)

- Pressure transducer in aorta (Konigsberg)
- Pacing lead (epicardial) + Pacemaker
- ECG-lead connected to one Konigsberg-Transducer
- Pressure transducer in left ventricle (Konigsberg)

3. Daily checks

Daily health checks were carried out throughout the study. Checks included: Skin buttons, Body weight, girth, Breathing rate (qualitative measures)

4. Weekly checks

Additional veterinary health checks were carried out weekly (General health, cardiovascular and respiratory assessment, skin buttons)

4. Lab studies

During the study the dogs were routinely tested on the following occasions:

- After post-surgery recovery period before pacing (1x)
- During pacing before drug treatment (1xweek = 2x)
- During treatment (2x/week = 6 x in 3 weeks)

The protocol took ca. 90 min and included

- Transport of animals to the lab
- Baseline measurements after switching off the pacemaker and blood sampling (30 min)
- Exercise on the treadmill (20 min)
- Recovery (10 min)
- Transport back with pacemaker switched on.

5. Dosing

Dosing of the animals during the 3-week treatment period took place BID @ 8 am and 4 pm:

| | AM (8:00 h) | PM (16:00 h) |
|---|---|---|
| Placebo | Furosemide tablet (20 mg) Vehicle-Gavage | Furosemide tablet (20 mg) Vehicle-Gavage |
| ACEI | Furosemide tablet (20 mg) E.-Gavage (0.5 mg/kg) | Furosemide tablet (20 mg) E.-Gavage (0.5 mg/kg) |
| ETra (Compound | Furosemide tablet (20 mg) X)Vehicle-Gavage | Furosemide tablet (20 mg) ETra-Gavage (2 mg/kg) |

Improvement in cardiac function is illustrated by the effect of Compound X on the left ventricular enddiastolic pressure (LVEDP) in resting dogs before and during progressive CHF induced by pacing at 210-240bpm. Test compounds were given for 3 weeks while pacing continued. In the graph below, the Y-axis is LVEDP in mm, and the X-axis is the time in weeks. Significant improvements over both placebo and enalapril were observed. [In the study, 5 dogs were treated with placebo, 3 dogs were treated with enalapril, and 5 dogs were treated with Compound X]

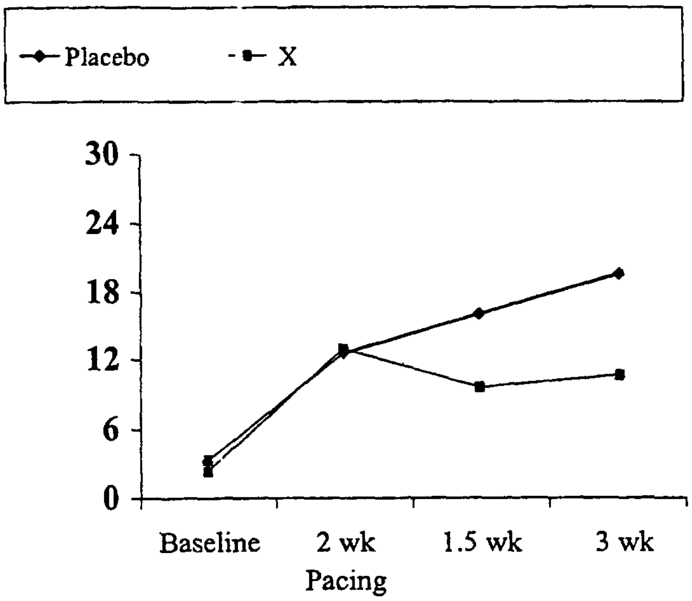


Dogs were paced for 2 weeks to develop CHF and then assigned to oral gavage treatment with either placebo (vehicle), Compound X (2 mg/kg/day, sid), or enalapril (1 mg/kg/day, bid). In addition, all animals received the diuretic furosemide (4 mg/kg/day, bid) throughout treatment period. During the 3-week treatment period, rapid ventricular pacing was continued. Pacing led to deterioration of myocardial and circulatory function with progression of CHF as indicated by increases in left ventricular enddiastolic pressure (LVEDP) and reductions of cardiac contractility and output. In addition, all animals were assessed for clinical parameters of CHF by an independent veterinarian. All dogs showed signs of left and right ventricular failure as indicated by pulmonary edema and mild to moderate ascites, respectively accompanied by compensatory weight losses.

Throughout the study dogs were studied twice weekly at rest and during standardised exercise on a treadmill at two different speeds. Compound X significantly improved LVEDP not only under resting conditions but even more so during treadmill exercise. Compound X lowered LVEDP below pretreatment values. This indicates that this endothelin receptor antagonist has the potential to improve the disease status of dogs with CHF.

Compound X's effects on systemic circulatory function were indicated by lower arterial blood pressure (BP) and maintained or even increased cardiac output.

The beneficial effects of Compound X on haemodynamic functions were paralleled by the clinical observation of the animals. While in the placebo group three out of six animals developed exercise intolerance and thus reached class III heart failure within the NYHA scale (I-IV), all animals treated with Compound X maintained exercise tolerance throughout the study and could therefore be assigned to the better heart failure class II.

With respect to treatment of cats, three cats were dosed both IV and oral at 2 mg/kg. The measured half-life was ca. 4.5 hours in cats. The total concentration at 24 hours after an oral dose of 2 mg/kg given to cats was ca. 232ng/ml.

The compounds of formula I will normally be administered orally or by any parenteral route in the form of a pharmaceutical preparation comprising the active ingredient, optionally in the form of a non-toxic organic, or inorganic, acid, or base, addition salt, in a pharmaceutically acceptable dosage form. Depending upon the disorder and patient to be treated, as well as the route of administration, the compositions may be administered at varying doses (see below).

The terms "pharmaceutical", pharmaceutically-acceptable, etc., are intended to include the corresponding veterinary equivalents where appropriate.

While it is possible to administer a compound of the invention directly without any formulation, the compounds are preferably employed in the form of a pharmaceutical, or veterinary, formulation comprising a pharmaceutically, or veterinarily, acceptable carrier, diluent or excipient and a compound of the invention. The carrier, diluent or excipient may be selected with due regard to the intended route of administration and standard pharmaceutical, and/or veterinary, practice. Pharmaceutical compositions comprising the compounds of the invention may contain from 0.1 percent by weight to 90.0 percent by weight of the active ingredient.

The methods by which the compounds may be administered for veterinary use include oral administration of a capsule, bolus, tablet, powder, drench , elixir, solution, paste, suspension, medicated feed or drink such as drinking water or buccal or sublingual formulations, which may contain flavouring, palatable and/or colouring agents, which may

include agents for immediate, delayed, modified, sustained, pulsed or controlled release, topical administration as an ointment, a pour-on, spot-on, dip, spray, mousse, shampoo, collar, ear tag or powder formulation, or, alternatively, they can be administered by injection (eg subcutaneously, intramuscularly or intravenously), or as an implant. Other possible routes of administration are intranasal, inhalations and suppository. Such formulations may be prepared in a conventional manner in accordance with standard veterinary practice.

For treatment of companion animals such as cats, dogs and horses, the compounds of formula I, or their veterinarily acceptable salts, can be administered alone but will generally be administered in admixture with a pharmaceutical / veterinary carrier selected with regard to the intended route of administration and standard pharmaceutical / veterinary practice. For example they can be administered orally in the form of tablets containing such excipients as starch or lactose or in capsules or ovules either alone or in admixture with excipients or in the form of elixirs, solutions or suspensions containing the substance in a liquid carrier, for example a vegetable oil, glycerine or water with a flavouring or colouring agent. They can be injected parenterally, for example intravenously, intramuscularly or subcutaneously. For parental administration, they are best used as sterile aqueous solutions which may contain other substances, for example, enough glucose or salts to make the solution isotonic with blood. For parenteral administration the substance may also be administered as a solution or suspension in a suitable oil, for example polyethylene glycol, lecithin or sesame oil.

The substances may also be administered through inhalation of a solution, suspension or emulsion that may be administered as a dry powder or in the form of an aerosol using a conventional propellant such as dichlorodifluoromethane.

Alternatively the substances can be administered in the form of a suppository or pessary, or they may be applied topically in the form of a lotion, solution, cream, ointment or dusting powder or in the form of a medicated plaster, patch or membrane. For example they may be incorporated in a cream containing an aqueous emulsion of polyethylene glycols or liquid paraffin. The compounds may also be administered intranasally.

The formulations will vary with regard to the weight of active compound contained therein, depending on the species of animal to be treated, the severity and type of infection and the body weight of the animal. For parenteral, topical and oral administration, typical dose ranges of the active ingredient are 0.01 to 100 mg per kg of body weight of the animal. Preferably the range is 0.1 to 10 mg per kg.

In any event, the veterinary practitioner, or the skilled person, will be able to determine the actual dosage which will be most suitable for an individual patient, which may vary with the species, age, weight and response of the particular patient. The above dosages are exemplary of the average case; there can, of course, be individual instances where higher or lower dosage ranges are merited, and such are within the scope of this invention.

As an alternative for treating animals, the compounds may be administered with the animal feedstuff and for this purpose a concentrated feed additive or premix may be prepared for mixing with the normal animal feed.

Compounds of formula I may be administered either alone or in combination with one or more agents used in the treatment or prophylaxis of disease or in the reduction or suppression of symptoms. Examples of such agents (which are provided by way of illustration and should not be construed as limiting) include antiparasitics, eg arylpyrazoles such as fipronil, lufenuron, imidacloprid, avermectins (eg abamectin, ivermectin, doramectin, selamectin), milbemycins, organophosphates, pyrethroids; antihistamines, eg chlorpheniramine, trimeprazine, diphenhydramine, doxylamine; antifungals, eg fluconazole, ketoconazole, itraconazole, griseofulvin, amphotericin B; antibacterials, eg enroflaxacin, marbofloxacin, ampicillin, amoxycillin; antiinflammatories eg prednisolone, betamethasone, dexamethasone, carprofen, ketoprofen; dietary supplements, eg gamma-linoleic acid; inotropic agents (e.g. digoxin), diuretic agents (e.g. furosemide), and emollients. Therefore, the invention further provides a product containing a compound of the invention and one or more selected compounds from the above list as a combined preparation for simultaneous, separate or sequential use in the treatment of diseases modulated *via* endothelin receptors

The skilled person will also appreciate that compounds of the invention may be taken as a single dose or on an "as required" basis (i.e. as needed or desired).

Such formulations are prepared in a conventional manner in accordance with standard pharmaceutical or veterinary practice. Thus capsules, boluses or tablets may be prepared by mixing the active ingredient with a suitable finely divided diluent or carrier additionally containing a disintegrating agent and/or binder such as starch, lactose, talc or magnesium stearate, etc. Oral drenches are prepared by dissolving or suspending the active ingredient in a suitable medium. Pour-on or spot-on formulations may be prepared by dissolving the active ingredient in an acceptable liquid carrier vehicle such as butyl digol, liquid paraffin or a non-volatile ester, optionally with the addition of a volatile component such as propan-2-ol.

Alternatively, pour-on, spot-on or spray formulations can be prepared by encapsulation, to leave a residue of active agent on the surface of the animal. Injectable formulations may be prepared in the form of a sterile solution which may contain other substances, for example enough salts or glucose to make the solution isotonic with blood. Acceptable liquid carriers include vegetable oils such as sesame oil, glycerides such as triacetin, esters such as benzyl benzoate, isopropyl myristate and fatty acid derivatives of propylene glycol, as well as organic solvents such as pyrrolidin-2-one and glycerol formal. The formulations are prepared by dissolving or suspending the active ingredient in the liquid carrier such that the final formulation contains from 0.1 to 10% by weight of the active ingredient.

These formulations will vary with regard to the weight of active substance contained therein, depending on the species of animal to be treated, the severity and type of infection and the body weight of the animal. For parenteral, topical and oral administration, typical dose ranges of the active ingredient are 0.01 to 100 mg per kg of body weight of the animal. Preferably the range is 0.1 to 10 mg per kg, more preferably 0.5 to 5 mg per kg, most preferably from 2 to 4 mg/kg, for example ca. 2 mg per kg or 4 mg per kg.

The compositions are preferably formulated in a unit dosage form, each dosage containing from about 1 to about 500 mg, more usually about 5 to about 300 mg, of the active ingredient. The term "unit dosage form" refers to physically discrete units suitable as unitary dosages for human subjects and other mammals, each unit containing a predetermined quantity of active material calculated to produce the desired therapeutic effect, in association with a suitable pharmaceutical carrier.

As an alternative for veterinary use the substances may be administered with animal feedstuff and for this purpose a concentrated feed additive or premix may be prepared for mixing with the normal animal feed.

Thus, the invention further provides a formulation containing a compound of formula I, as defined above, or a veterinarily acceptable salt thereof, characterized in that it is adapted for administration to a companion animal. Suitable formulations include those adapted for oral administration, having a taste attractive to the companion animal (for example containing a suitable flavouring agent).

Preferably the route of administration is oral.

Preferably the formulation is a flavoured or unflavoured tablet.

Preferably the dosage given to the companion animal is about 2 to about 4mg/kg, i.e. for a 10kg animal the unit dosage contains about 20 to about 40 mg of the compound of formula I.

A preferred embodiment is that in which the compound of formula I is administered with a further active agent selected from an inotropic agent (e.g. digoxin), and a diuretic agent (e.g. furosemide). The co-administration can be sequential or simultaneous, and the routes of administration of the separate active agents can be different or they can be the same.

## Claims

1. The use of a compound of formula I,

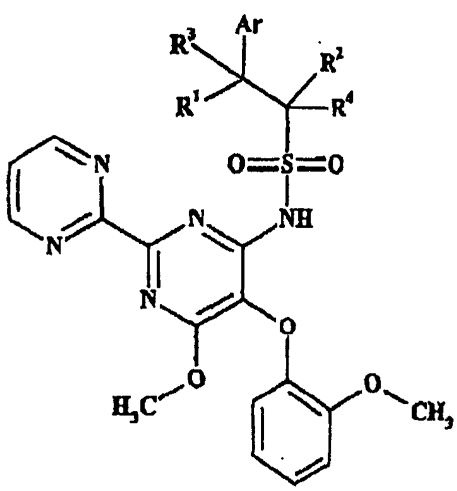

I

wherein $R^1$ and $R^2$ each represent H, or together represent a second carbon-carbon bond between the carbon atoms to which they are attached;

when $R^1$ and $R^2$ each represent H, then $R^3$ and $R^4$ also represent H;

when $R^1$ and $R^2$ together represent a second carbon-carbon bond between the carbon atoms to which they are attached, then $R^3$ and $R^4$ Independently represent H or $C_{1-6}$ alkyl;

Ar represents:

phenyl or naphthyl, which groups are optionally substituted by one or more groups selected from $C_{1-6}$ alkyl [which may itself be substituted by one or more substituents selected from halo, $C_{1-6}$ alkoxy, $CO_2H$, $NH_2$, NH

($C_{1-6}$ alkyl) and N($C_{1-6}$ alkyl$)_2$], halo, $C_{1-6}$ alkoxy, $CO_2H$, $C_{1-6}$ alkoxycarbonyl, $NO_2$, CN, $NH_2$, NH($C_{1-6}$ alkyl), N($C_{1-6}$ alkyl$)_2$, OH and $C_{1-3}$ alkylenedioxy, or
a 5- or 6-membered heteroaryl ring containing up to 4 heteroatoms selected from N, 0 and S, which group is optionally substituted by one or more groups selected from $C_{1-6}$ alkyl [which may itself be substituted by one or more substituents selected from halo, $C_{1-6}$ alkoxy, $CO_2H$, $NH_2$, NH($C_{1-6}$ alkyl) and N($C_{1-6}$ alkyl$)_2$], halo, $C_{1-6}$ alkoxy, $CO_2H$, $C_{1-6}$ alkoxycarbonyl, $NO_2$, CN, $NH_2$, NH($C_{1-6}$ alkyl) and N($C_{1-6}$ alkyl$)_2$;

or a veterinarily acceptable salt thereof;
in the manufacture of a medicament for the treatment or prophylaxis of an endothelin-mediated disorder in a companion animal.
wherein the companion animal is a cat, a dog or a horse.

**2.** The use as claimed in claim 1, wherein the endothelin-mediated disorder is hypertension, congestive heart failure or chronic renal failure.

**3.** The use as claimed In any one of the preceding claims, wherein $R^1$ and $R^2$ each represent H.

**4.** The use as claimed in any one of the preceding claims, wherein $R^3$ and $R^4$ each represent H.

**5.** The use as claimed in any one of the preceding claims, wherein Ar represents phenyl, naphthyl or thienyl, which groups are optionally substituted by one or more groups selected from $C_{1-6}$ alkyl, halo, $CF_3$, $C_{1-6}$ alkoxy, $CO_2H$ and $C_{1-6}$ alkoxycarbonyl;

**6.** The use as claimed in any one of the preceding claims, wherein Ar is phenyl.

**7.** The use as claimed in any one of the preceding claims, wherein the use is for manufacturing a medicament for the treatment of congestive heart failure in a dog.

**8.** The use as claimed in any one of claims 1 to 6, wherein the use is for manufacturing a medicament for the treatment of chronic renal failure in a cat.

**9.** A formulation containing a compound of formula I, as defined in claim 1, or a veterinarily acceptable salt thereof, **characterized in that** it is adapted for administration to a cat, dog or horse.

**10.** A formulation as claimed in claim 9, which is adapted for oral administration and has a taste attractive to the cat, dog or horse.

**11.** A pharmaceutical pack comprising a formulation according to claim 9 or 10 and instructions describing the treatment of congestive heart failure in a dog.

**12.** A pharmaceutical pack comprising a formation according to claim 9 or 10 and instructions describing the treatment of chronic renal failure in a cat.

## Patentansprüche

**1.** Verwendung einer Verbindung der Formel I,

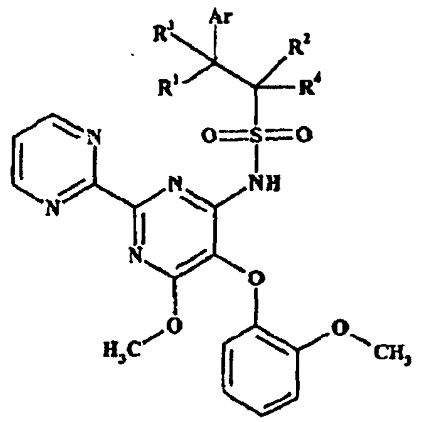

I

worin $R^1$ und $R^2$ jeweils H wiedergeben, oder zusammen eine zweite Kohlenstoff-Kohlenstoff-Bindung zwischen den Kohlenstoffatomen, an die sie gebunden sind, wiedergeben;

wenn $R^1$ und $R^2$ jeweils H wiedergeben, dann $R^3$ und $R^4$ auch H wiedergeben;

wenn $R^1$ und $R^2$ zusammen eine zweite Kohlenstoff-Kohlenstoff-Bindung zwischen den Kohlenstoffatomen, an die sie gebunden sind, wiedergeben, dann $R^3$ und $R^4$ unabhängig H oder $C_{1-6}$-Alkyl wiedergeben;

Ar wiedergibt:

Phenyl oder Naphthyl, wobei die Gruppen gegebenenfalls mit einer oder mehreren Gruppen, ausgewählt aus $C_{1-6}$-Alkyl [das selbst mit einem oder mehreren Substituenten, ausgewählt aus Halogen, $C_{1-6}$-Alkoxy, $CO_2H$, $NH_2$, $NH(C_{1-6}$-Alkyl) und $N(C_{1-6}$-Alkyl$)_2$, substituiert sein kann], Halogen, $C_{1-6}$-Alkoxy, $CO_2H$, $C_{1-6}$-Alkoxycarbonyl, $NO_2$, CN, $NH_2$, $NH(C_{1-6}$-Alkyl), $N(C_{1-6}$-Alkyl$)_2$, OH und $C_{1-3}$-Alkylendioxy, substituiert sind, oder einen 5- oder 6-gliedrigen Heteroarylring, der bis zu 4 Heteroatome, ausgewählt aus N, 0 und S, enthält, wobei die Gruppe gegebenenfalls mit einer oder mehreren Gruppen, ausgewählt aus $C_{1-6}$-Alkyl [die selbst mit einem oder mehreren Substituenten, ausgewählt aus Halogen, $C_{1-6}$-Alkoxy, $CO_2H$, $NH_2$, $NH(C_{1-6}$-Alkyl) und $N(C_{1-6}$-Alkyl$)_2$, substituiert sein kann], Halogen, $C_{1-6}$-Alkoxy, $CO_2H$, $C_{1-6}$-Alkoxycarbonyl, $NO_2$, CN, $NH_2$, $NH(C_{1-6}$-Alkyl) und $N(C_{1-6}$-Alkyl$)_2$, substituiert ist;

oder eines veterinär verträglichen Salzes davon;

bei der Herstellung eines Arzneimittels für die Behandlung oder Prophylaxe einer Endothelin-vermittelten Störung bei einem Haustier, wobei das Haustier eine Katze, ein Hund oder ein Pferd ist.

**2.** Verwendung nach Anspruch 1, worin die Endothelinvermittelte Störung Hypertension, Herzstauungsinsuffizienz oder chronisches Nierenversagen ist.

**3.** Verwendung nach einem beliebigen der vorangehenden Ansprüche, worin $R^1$ und $R^2$ jeweils H wiedergeben.

**4.** Verwendung nach einem beliebigen der vorangehenden Ansprüche, worin $R^3$ und $R^4$ jeweils H wiedergeben.

**5.** Verwendung nach einem beliebigen der vorangehenden Ansprüche, worin Ar Phenyl, Naphthyl oder Thienyl wiedergibt, wobei die Gruppen gegebenenfalls mit einer oder mehreren Gruppen, ausgewählt aus $C_{1-6}$-Alkyl, Halogen, $CF_3$, $C_{1-6}$-Alkoxy, $CO_2H$ und $C_{1-6}$-Alkoxycarbonyl, substituiert sind.

**6.** Verwendung nach einem beliebigen der vorangehenden Ansprüche, worin Ar Phenyl darstellt.

**7.** Verwendung nach einem beliebigen der vorangehenden Ansprüche, wobei die Verwendung zur Herstellung eines Arzneimittels für die Behandlung von Herzstauungsinsuffizienz bei einem Hund erfolgt.

**8.** Verwendung nach einem beliebigen der Ansprüche 1 bis 6, wobei die Verwendung zur Herstellung eines Arzneimittels für die Behandlung von chronischem Nierenversagen bei einer Katze erfolgt.

**9.** Formulierung, enthaltend eine Verbindung der Formel I, wie in Anspruch 1 definiert, oder ein veterinär verträgliches Salz davon, **dadurch gekennzeichnet, dass** sie zur Verabreichung an eine Katze, einen Hund oder ein Pferd angepasst ist.

**10.** Formulierung nach Anspruch 9, die zur oralen Verabreichung angepasst ist und die einen Geschmack aufweist, der für die Katze, den Hund oder das Pferd attraktiv ist.

**11.** Pharmazeutische Packung, umfassend eine Formulierung nach Anspruch 9 oder 10 und Anweisungen, die die Behandlung von Herzstauungsinsuffizienz bei einem Hund beschreiben.

**12.** Pharmazeutische Packung, umfassend eine Formulierung nach Anspruch 9 oder 10 und Anweisungen, die die Behandlung von chronischem Nierenversagen bei einer Katze beschreiben.

## Revendications

**1.** Utilisation d'un composé de formule I

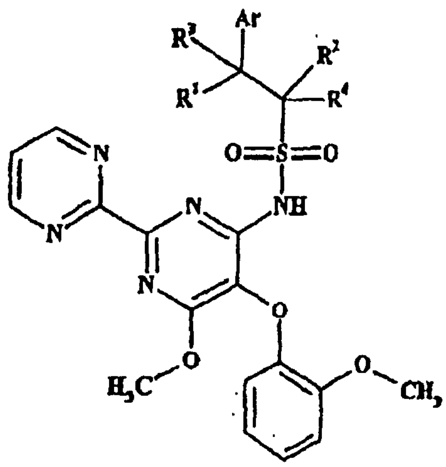

I

dans laquelle $R^1$ et $R^2$ représentent chacun H, ou bien, conjointement, représentent une seconde liaison carbone-carbone entre les atomes de carbone auxquels ils sont fixés ;

lorsque $R^1$ et $R^2$ représentent chacun H, alors $R^3$ et $R^4$ représentent également H ;

lorsque $R^1$ et $R^2$, conjointement, représentent une seconde liaison carbone-carbone entre les atomes de carbone auxquels ils sont fixés, alors $R^3$ et $R^4$ représentent indépendamment H ou des groupes alkyle en $C_1$ à $C_6$ ;

Ar représente :

un groupe phényle ou naphtyle, ces groupes étant facultativement substitués avec un ou plusieurs groupes choisis entre des groupes alkyle en $C_1$ à $C_6$ [qui peut lui-même être substitué avec un ou plusieurs substituants choisis entre des substituants halogéno, alkoxy en $C_1$ à $C_6$, $CO_2H$, $NH_2$, NH(alkyle en $C_1$ à $C_6$) et N(alkyle en $C_1$ à $C_6$)$_2$], halogéno, alkoxy en $C_1$ à $C_6$ , $CO_2H$, (alkoxy en $C_1$ à $C_6$)carbonyle, $NO_2$, CN, $NH_2$, NH (alkyle en $C_1$ à $C_6$), N(alkyle en $C_1$ à $C_6$ )$_2$, OH et alkylènedioxy en $C_1$ à $C_3$, ou

un noyau hétéroaryle penta- ou hexagonal contenant jusqu'à 4 hétéroatomes choisis entre N, O et S, groupe qui est facultativement substitué avec un ou plusieurs groupes choisis entre des groupes alkyle en $C_1$ à $C_6$ [qui peut lui-même être substitué avec un ou plusieurs substituants choisis entre des substituants halogéno, alkoxy en $C_1$ à $C_6$, $CO_2H$, $NH_2$, NH (alkyle en $C_1$ à $C_6$) et N (alkyle en $C_1$ à $C_6$)$_2$], halogéno, alkoxy en $C_1$ à $C_6$, $CO_2H$, (alkoxy en $C_1$ à $C_6$)carbonyle, $NO_2$, CN, $NH_2$, NH(alkyle en $C_1$ à $C_6$) et N (alkyle en $C_1$ à $C_6$)$_2$ ;

ou d'un de ses sels acceptables du point de vue vétérinaire ;

dans la production d'un médicament destiné au traitement ou à la prophylaxie d'un trouble à médiation par une endothéline chez un animal de compagnie.

**2.** Utilisation suivant la revendication 1, dans laquelle le trouble à médiation par une endothéline est l'hypertension, l'insuffisance cardiaque congestive ou l'insuffisante rénale chronique.

**3.** Utilisation suivant l'une quelconque des revendications précédentes, dans laquelle $R^1$ et $R^2$ représentent chacun H.

**4.** Utilisation suivant l'une quelconque des revendications précédentes, dans laquelle $R^3$ et $R^4$ représentent chacun H.

**5.** Utilisation suivant l'une quelconque des revendications précédentes, dans laquelle Ar représente un groupe phényle, naphtyle ou thiényle, ces groupes étant facultativement substitués avec un ou plusieurs groupes choisis entre des groupes alkyle en $C_1$ à $C_6$, halogéno, $CF_3$, alkoxy en $C_1$ à $C_6$, $CO_2H$ et (alkoxy en $C_1$ à $C_6$)carbonyle.

**6.** Utilisation suivant l'une quelconque des revendications précédentes, dans laquelle Ar représente un groupe phényle.

**7.** Utilisation suivant l'une quelconque des revendications précédentes, qui est destinée à la production d'un médicament pour le traitement de l'insuffisance cardiaque congestive chez un chien.

**8.** Utilisation suivant l'une quelconque des revendications 1 à 6, qui est destinée à la production d'un médicament pour le traitement de l'insuffisance rénale chronique chez un chat.

**9.** Formulation contenant un composé de formule 1, tel que défini dans la revendication 1, ou un de ses sels acceptables du point de vue vétérinaire, **caractérisée en ce qu'**elle est adaptée à l'administration à un chat, un chien ou un cheval.

**10.** Formulation suivant la revendication 9, qui est adaptée à l'administration orale et qui a un goût agréable pour le chat, le chien ou le cheval.

**11.** Emballage pharmaceutique comprenant une formulation suivant la revendication 9 ou 10 et des instructions décrivant de l'insuffisance cardiaque congestive chez un chien.

**12.** Emballage pharmaceutique comprenant une formulation suivant la revendication 9 ou 10 et des instructions décrivant le traitement de l'insuffisance rénale chronique chez un chat.